# EUROPEAN PATENT SPECIFICATION

(11) **EP 3 740 194 B1**
(45) Date of publication and mention of the grant of the patent: **09.04.2025**
(21) Application number: 19740805.7
(22) Date of filing: 17.01.2019
(51) Int. Cl.: A61K 31/46, A61K 9/00, A61P 27/10, A61M 11/00, A61B 5/00, A61M 15/02, A61F 9/00

(54) **METHODS AND DEVICES FOR DELIVERING ATROPINE TO THE EYE AS A MICRO-DOSE STREAM OF DROPLETS**
VERFAHREN UND VORRICHTUNGEN ZUR ABGABE VON ATROPIN IN DAS AUGE ALS MIKRODOSIERSTROM VON TRÖPFCHEN
MÉTHODES ET DISPOSITIFS POUR ADMINISTRER DE L'ATROPINE À UN OEIL SOUS LA FORME D'UN FLUX MICRODOSÉ DE GOUTTELETTES

(30) Priority: 17.01.2018 US 201862618348 P
(43) Date of publication of application: 25.11.2020
(73) Proprietor: Eyenovia, Inc., New York, New York 10017 (US)
(72) Inventor: IANCHULEV, Tsontcho, New York, New York 10017 (US); CLAUSON, Luke, New York, New York 10017 (US)
(74) Representative: Mewburn Ellis LLP
(86) International application number: PCT/US2019/013944
(87) International publication number: WO 2019/143777

(56) References cited:
- JP-A- 2007 308 398
- US-A1- 2012 143 152
- US-A1- 2016 338 947
- US-A1- 2017 344 714
- US-B1- 6 425 888
- BARATHI V A ET AL: "Effects of unilateral topical atropine on binocular pupil responses and eye growth in mice", VISION RESEARCH, ELSEVIER, AMSTERDAM, NL, vol. 49, no. 3, 1 February 2009 (2009-02-01), pages 383 - 387, XP025914419, ISSN: 0042-6989, [retrieved on 20090201], DOI: 10.1016/J.VISRES.2008.11.005
- PINELES STACY L ET AL: "Atropine for the Prevention of Myopia Progression in Children A Report by the American Academy of Ophthalmology", OPHTHALMOLOGY, vol. 124, no. 12, December 2017 (2017-12-01), pages 1857 - 1866, XP085266078, ISSN: 0161-6420, DOI: 10.1016/J.OPHTHA.2017.05.032
- NAITO TOMOKO ET AL: "Comparison of success rates in eye drop instillation between sitting position and supine position", PLOS ONE, vol. 13, no. 9, 20 September 2018 (2018-09-20), pages e0204363, XP093051476, DOI: 10.1371/journal.pone.0204363
- PATEL SANTOSH C ET AL: "Compliance in Patients Prescribed Eyedrops for Glaucoma", OPHTHALMIC SURGERY, LASERS AND IMAGING RETINA, vol. 26, no. 3, 1 May 1995 (1995-05-01), pages 233 - 236, XP093051471, ISSN: 2325-8160, DOI: 10.3928/1542-8877-19950501-14
- WINFIELD A. J. ET AL: "A study of the causes of non-compliance by patients prescribed eyedrops.", BRITISH JOURNAL OF OPHTHALMOLOGY, vol. 74, no. 8, 1 August 1990 (1990-08-01), GB, pages 477 - 480, XP093051469, ISSN: 0007-1161, DOI: 10.1136/bjo.74.8.477
- MATSUI DOREEN M.: "DRUG COMPLIANCE IN PEDIATRICS", PEDIATRIC CLINICS OF NORTH AMERICA., vol. 44, no. 1, 1 February 1997 (1997-02-01), GB, pages 1 - 14, XP093051477, ISSN: 0031-3955, DOI: 10.1016/S0031-3955(05)70459-4
- JR POLLING ET AL: "Effectiveness study of atropine for progressive myopia in Europeans", EYE,, vol. 30, 22 April 2016 (2016-04-22), pages 998 - 1004, XP002787205

## Description

### FIELD OF THE INVENTION

The present disclosure relates to droplet delivery devices, methods of administration and uses thereof, specifically for the administration of droplets of a composition comprising atropine to the eye in order to treat myopia in a child, as defined in more detail in the claims.

Any reference to a method of treatment of the human or animal body by therapy involving a certain compound or composition is to be interpreted as a reference to said compound or composition for use in said method of treatment.

The invention is defined in the claims. Any subject-matter beyond the scope of the claims is not part of the invention and provided for comparison or reference purposes only.

### BACKGROUND OF THE INVENTION

Atropine formulations have been used in ophthalmology for pupil dilation and for amblyopia treatment. In addition, recent studies suggest that atropine may be used for the treatment of Myopia. Recent academic studies have suggested that some side effects associated with use of the standard 1% concentration may be avoided by using lower concentrations than of the active ingredient. These studies indicate that 1% atropine drops are not as well tolerated as lower concentrations, e.g., 0.1% and 0.01%. The lower concentration formulations are typically prepared by simply diluting the 1% atropine solution. However, these low volume solutions are typically unstable for long term use.

Moreover, a typical medical droplet as dispensed by an eye dropper bottle can vary, depending on the viscosity and surface tension of the fluid. In order to control the amount of active ingredient that is administered in a single droplet, the concentration of the active ingredient is adjusted by volume. Once the concentration is defined, a correct dosage may require one drop or more. However, since the human eye can typically retain only 7 µl of fluid at a time, even a single medical droplet can result in overflow and loss of part of the medication from the eye. Multiple drop dosage often compounds the problem of medication retention in the eye. Subjects will typically administer all droplets required for a dosage in one sitting, which exacerbates the problem and can result in 50 to 90% of the medication overflowing and leaking out of the eye.

Given the above and other limitations of current ophthalmic delivery, a need exists for an efficient delivery system for solutions to the eye, including solutions containing medicaments such as atropine.

Pineles Stacy L et al. ("Atropine for the Prevention of Myopia Progression in Children A Report by the American Academy of Ophthalmology", OPHTHALMOLOGY, vol. 124, no. 12, pages 1857-1866) is a report about atropine for the prevention of myopia progression in children.

US6425888B1 discloses a device for delivering a unit dose of treatment fluid as jet or droplets, in particular for ocular use, which minimises wastage of fluid and eliminates the need for preservatives in ophthalmic treatment fluids.

### SUMMARY OF THE INVENTION

In certain aspects, the disclosure provides method for delivering a composition comprising atropine to an eye of a child in need thereof as a micro-dose stream of droplets.

The method generally includes, (a) generating a micro-dose stream of droplets including the composition comprising atropine via a piezoelectric droplet delivery device, wherein the micro-dose stream of droplets has an average ejected droplet diameter 20 microns to 100 microns; and (b) delivering the micro-dose stream of droplets to the eye of said child. The invention treats myopia, in particular progressive myopia (nearsightedness) in children in need thereof.

In certain aspects, the piezoelectric droplet delivery device comprises an ejector mechanism, the ejector mechanism comprising a generator plate and a piezoelectric actuator, wherein the generator plate includes a plurality of openings formed through its thickness; and wherein the piezoelectric actuator is operable to directly or indirectly oscillate the generator plate, at a frequency to generate a directed stream of droplets of said low dosage volume medicament composition.

The micro-dose stream of droplets delivered to the eye of the subject is from 3 microliters to less than 10 microliters. The micro-dose stream of droplets has an average initial ejecting velocity about of at least 3 m/s to about 4 m/s. In some embodiments, the micro-dose stream of droplets is delivered to the eye of the subject in less than 80 ms.

In certain aspects, the composition comprises atropine at a concentration of at least 0.5 % by weight, at least 0.8 % by weight, 1% or more by weight, 0.5 to 1.2% by weight, etc.

In yet other aspects, the piezoelectric droplet delivery device is oriented within 25°of horizontal during generation and delivery of the micro-dose stream of droplets.

These and other features will become apparent from the following description of the preferred embodiments, claims and drawings.

### DETAILED DESCRIPTION

The present disclosure provides methods and devices which improve upon prior art methods and devices for delivering atropine to the eye. Specifically, the present disclosure utilize relatively high concentrations of atropine in the solution to be administered, contrary to current trends, but delivers a much smaller dose to a subject, which thereby reduces, and even prevents, excess fluid from being unabsorbed by the target tissues.

Maintaining higher concentrations of atropine in the solution to be administered may also reduce or avoid issues with the instability of lower concentration solutions.

The invention provides a method for delivering a composition comprising atropine to an eye of a child in need thereof as a micro-dose stream of droplets. The method generally includes, (a) generating a micro-dose stream of droplets including the composition comprising atropine via a piezoelectric droplet delivery device, wherein the micro-dose stream of droplets has an average ejected droplet diameter of 20 microns to 100 microns; and (b) delivering the micro-dose stream of droplets to the eye of said child. The method treats myopia, in particular progressive myopia (nearsightedness) in children in need thereof.

In certain aspects, the present methods and devices utilize relatively high concentrations of atropine to avoid the stability problems of low concentration formulations currently favored.

The invention provides a piezoelectric droplet delivery device configured to achieve micro-dosing that is many times more precise than conventional eye droppers. In certain aspects, the micro-dosing delivers doses of 6-8 µL in a targeted manner, directly coating the corneal surface (where 80% of intraocular drug penetration occurs) rather than the conjunctiva of the eye of the subject.

Without intending to be limited by theory, it is believed that focusing a significant portion (i.e., over 50%, 60%, 70%, 80%, etc.) of the active agent to be administered to the eye of the subject directly to the corneal surface rather than the conjunctiva reduces collateral tissue exposure. In this regard, it is believed that administration directly to the corneal surface will reduce greater than 75% of the active agent's potential systemic exposure, to thereby reduce toxicity and result in potentially gentler and more tolerable treatments. In other aspects, the micro-therapeutic approach of the disclosure also reduces waste associated with conventional eye droppers.

In yet other aspects, advantages of the micro-dosing approach of the disclosure includes:
**Dose reduction:** micro-dosing achieves precise volumetric control at the microliter level to deliver 6-8 µL, which is the physiologic capacity of the tear film, e.g., as compared to an eye dropper pipette macro-dose of 30-50 µL which results in overdosing, ocular toxicity and systemic leaching into the plasma.

**Targeted dose instillation:** the piezoelectric droplet delivery device described herein allows for targeted delivery to the ocular surface and cornea, avoiding the conjunctival cul-de-sac. The droplet micro-jet created by the piezoelectric vibrations is columnated and focused to provide accurate delivery to the corneal surface where the majority of ocular penetration occurs. Additionally, in certain embodiments, the device may include an LED targeting mechanism to allow proper positioning and objective alignment.

**Speed of delivery:** unlike a simple aerosolized mechanism, the piezoelectric droplet delivery device provides micro-droplet ejection control that creates a fast and targeted micro-jet delivery that provides ejected droplets to the ocular surface in less than 80 milliseconds, beating the eye's 100 millisecond blink reflex.

**Smart electronics:** in certain embodiments, the piezoelectric droplet delivery device includes smart electronics and mobile e-health technology are designed to track when a patient administers treatment. This enables physicians to monitor patient compliance accurately. In certain aspects, this technology will improve compliance and chronic disease management by empowering patients and physicians with access to dynamic, real time monitoring and compliance data for a more intelligent and personalized therapeutic paradigm.

In other aspects, the disclosure generally relates to piezoelectric droplet delivery devices useful, *e.g.,* in the delivery of a directed stream of droplets for ophthalmic use, more particularly, for use in the delivery of ophthalmic fluid to the eye. Droplets may be formed by an ejector mechanism from fluid contained in a reservoir coupled to the ejector mechanism. Except as otherwise described herein, the ejector mechanism and reservoir may be disposable or reusable, and the components may be packaged in a housing of an ejector device.

Devices are provided and methods are described for the reproducible delivering a therapeutically effective low volume micro-dose of a composition to a desired target (e.g., an eye of a subject in need thereof, as compared to standard eye dropper use and dosage volume). In certain aspects, the low volume micro-dose of a composition comprises atropine at a concentration of at least 0.5% by weight, at least 0.7% by weight, at least 0.8 % by weight, 1% by weight, etc. In certain aspects, the devices and methods are for the treatment of myopia, in particular progressive myopia (nearsightedness) inchildren. In certain aspects, the therapeutically effective low volume micro-dose of a composition may be delivered to an eye at, *e.g.,* 3/4, 1/2, 1/4, 1/6, 1/8, (e.g., ~0.02-0.75) etc. of the volume of a standard eye dropper volume. By way of example, in certain embodiments, from from microliters to 10 microliters of a micro-dose of a composition may be delivered to the eye of a subject, as compared to approximately 25 µl to approximately 70 µl by way of a standard eye dropper, while obtaining equivalent or improved therapeutic efficacy.

Dosing strategies also may incorporate various approaches to initiating treatment, stopping treatment, switching treatment and responding to different subject states. Examples of dosing modes or strategies include once-a-day dosing, twice-a-day dosing, three times-a-day dosing, continuous dosing, bolus dosing, weekly dosing, monthly dosing, taper dosing, need-based dosing, and feedback dosing by a physician, provider, subject, or family. In addition, dosing schemes may include dosing per eye, as needed. The clinical scenarios where these can be employed include chronic disease, disease exacerbation, need for suppression treatment, need for recurrence treatment, or state of treatment like medicament tolerance.

One embodiment discloses a method of delivering a therapeutically effective low volume micro-dose of a composition to an eye of a subject in need thereof, e.g., for the treatment of myopia, in particular progressive myopia (nearsightedness) in children, as compared to dosage volume of a standard eye dropper, the method comprising:
(a) generating a directed stream of droplets of the low volume micro-dose of a composition, wherein the droplets have a desired average drop size and average initial ejecting velocity; and
(b) delivering a therapeutically effective amount of the droplets of the low volume micro-dose of the composition to the eye of the subject, wherein the droplets deliver a desired percentage of the ejected mass of the droplets to the eye. Again, in certain aspects, the low volume micro-dose of a composition comprises atropine at a concentration of at least 0.5% by weight, at least 0.7% by weight, at least 0.8 % by weight, 1% by weight, from 0.5 to 1.2% by weight, etc.

Devices capable of providing and delivering therapeutically effective low volume micro-dose of the compositions to the eye are described herein. By way of example, the droplet delivery device may include a housing that comprises a fluid reservoir in fluid communication with an ejector mechanism. The directed stream of droplets may be generated via the ejector mechanism, the ejector mechanism comprising a generator plate and a piezoelectric actuator, wherein the generator plate includes a plurality of openings formed through its thickness. The piezoelectric actuator may be operable to directly or indirectly oscillate the generator plate, at a frequency to generate a directed stream of droplets of the low volume micro-dosing composition.

Without limitation, the droplet delivery device may be as described in US 8,684,980 or WO 2018/227190.

In one embodiment, the device further includes a fluid enclosure system to facilitate ejection of the stream of droplets. In such an embodiment, the fluid to be delivered to the eye is contained in an enclosure which holds the fluid to be dispensed in a chamber defined by the enclosure. The enclosure holds the dose of fluid in proximity to the openings of the generator plate of the ejector mechanism so that the fluid may be ejected in a short amount of time and with little residual volume left.

The enclosure has a lip positioned adjacent the generator plate. The lip may be unattached to the generate plate, but still in contact with the generator plate or may be spaced apart a short distance so that surface tension holds the fluid in the chamber. The generator plate may have a relatively small maximum amplitude when vibrated which is less than an average separation distance between the lip and the generator plate or less than a minimum separation distance between the lip and the generator plate.

The enclosure may also be shaped to cooperate with the generator plate to avoid capillary feed near the openings in the generator plate. To this end, the enclosure may be spaced apart from the generator plate so that at least 75%, at least 95%, or all of the openings are spaced at least 0.014 from the nearest part of the enclosure. The enclosure may also shaped so that all of the fluid can reach the openings of the generator plate in a short period of time. The enclosure may be configured with an internal surface shape in contact with the fluid such that at least 75%, at least 95%, or even all, of the internal surface is no more than 0.060 inch, or no more than 0.040 inch, from a nearest of the plurality of openings of the generator plate. Stated another way, the enclosure has an internal surface shaped such that the chamber is formed with at least 75%, at least 95%, or all, of the internal surface having direct line of sight to at least one of the openings of the generator plate. The inner surface of the enclosure may be hydrophobic over at least 70% of the inner surface in contact with fluid.

The lip may be biased against the generator plate with a modest force to prevent the fluid from escaping while not overly dampening vibrations. The lip may exert a force of no more than 3 gram-f on the vibrating element measured in the direction of a central axis of the vibrating element. The lip may also apply a spring load to the generator plate so that minor displacements due to temperature, pressure or shock from an impact (dropped) can be accommodated. The spring load may also help to address manufacturing tolerances which affect the load applied by the lip to the generator plate. The lip may exert a spring load on the generator plate with an average spring constant of no more than 60 gram-f/mm for displacements up to 0.050 mm. The enclosure itself may be resilient with a wall of the enclosure having a tapered portion with a relatively thin wall to provide flexibility. The tapered portion of the wall and has a ratio of radial displacement to longitudinal displacement of at least 1 to 3, at least 1 to 2 and may be at least 1 to 1. Stated another way, the tapered portion also extends radially with respect to the open end of the enclosure for at least half of an effective radius of the open end of the enclosure. The lip and/or the vibrating element may have a PTFE coating adjacent to the other to reduce friction therebetween. The coating(s) may extending around at least 270 degrees when viewed along the central axis.

The enclosure may allow air into to replace ejected fluid through the openings of the generator plate and/or between the lip and the generator plate and may include no dedicated vent opening. The maximum amplitude may be somewhat small which permits air to enter the chamber while still preventing fluid from escaping from the chamber. The enclosure to generator plate interface defines an enclosed border (which may be defined by either the generator plate or the enclosure) which is somewhat larger than the extent of the openings with an excess area which extends radially outwardly at least 0.3 times the effective radius of the enclosed feed area.

The enclosure may include a wall opening through the wall which exposes the chamber through the wall. The wall opening. The wall opening extends through the wall to expose the chamber through the wall without permitting fluid to escape while permitting air to enter when fluid is ejected. The wall opening has a longitudinal dimension measured from the lip in the direction of the central axis and a radial dimension measured in a radial direction relative to the central axis. The enclosure also has an internal wall with a side facing the openings in the generator plate. The longitudinal dimension of the wall opening is at least 80% of a separation distance between the generator plate and the side of the enclosure facing the openings. The radial dimension of the wall opening may be no more than 10%, or no more than 5%, of a circumference of the lip.

The wall opening tapers as it extends proximally away from the lip. The wall opening extends from the lip proximally and a circumferential dimension of the wall opening tapers down as the wall opening extends proximally from the lip. The wall opening tapers so that a tapered shape is oriented in the direction of the fluid inlet to the enclosure when viewed along the central axis. The wall opening may also extend through the frustoconical portion of the wall and may extend proximally from the lip for at least 80% of the length of the frustoconical portion.

The fluid may be delivered rapidly and at relatively high velocity and pressure to encourage all of the fluid to gather in the chamber. The total downstream volume of the fluid path from a pump or valve which isolates the chamber may be sized somewhat larger than the volume to permit the fluid to move within the enclosure somewhat and coalesce into a single droplet due to surface tension. The volume of the fluid may be 40%-70% of the total downstream volume.

The enclosure may also split the fluid flow into at least two (and may be three, four or more) inlets to the chamber. Each of the inlets directs the fluid at a sidewall before being directed at the plurality of openings of the generator plate. The enclosure has a main inlet which directs the flow in a direction within 30 degrees of the central axis while the inlets to the chamber are oriented 60-90 degrees from the central axis and directed at the sidewall. The enclosure may be an integrally formed structure which defines the chamber.

The pump may have a first part and a second part which reciprocate between a stored position, to a forward stroke position and back to the stored position in a single cycle. A cavity is formed between the two parts in which the fluid is drawn and subsequently expelled into the chamber. An air make-up chamber may also be coupled to the pump to force air into a fluid reservoir during each cycle to actively vent the fluid reservoir.

It is understood that the droplet delivery device may have be separated into reusable and disposable portions in innumerable different combinations. For example, the enclosure may form part of a reusable device together with the ejector mechanism, or may be disposable with the reservoir without departing from the scope of the invention.

In certain aspects, the stream of droplets may be generated by devices described herein in a controllable distribution of sizes, each distribution having an average droplet size. The average droplet size is in the range of 20 microns to 100 microns.

The device may also deliver the fluid at relatively high velocity which helps the fluid "stick" to the target tissue which, in this case, is corneal tissue resulting in direct corneal surface coating with the fluid. Eye droppers, on the other hand, do not have the ability to target a particular area of the eye and deliver the fluid at high velocity. Eye drops are large enough to migrate and seep into undesirable areas after delivery. In this regard, the droplets have an average initial ejecting velocity of 3 m/s to about 4 m/s. As used herein, the ejecting size and the ejecting initial velocity are the size and velocity of the droplets when the droplets leave the ejector plate. The stream of droplets directed at a target will result in deposition of a percentage of the mass of the droplets including their composition onto the desired location.

In certain aspects of the disclosure, the ejector devices will eject droplets without substantial evaporation, entrainment of air, or deflection off a target surface (*e.g.,* the surface of an eye), which facilitates consistent dosing. Average ejecting droplet size and average initial ejecting velocity are dependent on factors including fluid viscosity, surface tension, ejector plate properties, geometry, and dimensions, as well as operating parameters of the piezoelectric actuator including its drive frequency. In some implementations, about 60% to about 100%, about 65% to about 100%, about 75% to about 100%, about 80% to about 100%, about 85% to about 100%, about 90% to about 100%, about 95% to about 100%, *etc.,* of the ejected mass of droplets are deposited on the surface of the eye, such deposition being repeatable independent of operating and use conditions.

The direction of flow of the stream of droplets may be horizontal, or any direction a user chooses to aim the actuation mechanism during use. In certain aspects, the device may be oriented substantially horizontal, e.g., within 5° of horizontal, 10° of horizontal, 15° of horizontal, 20° of horizontal, 25° of horizontal, etc., and may aid the user in aiming the device. For example, a light, mirror or other visual alignment feature may be used as is known in the art. Horizontal delivery with alignment assistance may also improve ease of use and repeatability compared to an eyedropper which has no "aiming" mechanism and the drop is too large to target just corneal tissue. In this regard, it has been found that the same amount of drug with horizontal targeted corneal delivery and coating achieves higher pharmacodynamic effect compared to same amount of drug given in an eyedropper.

Droplet performance is generally related to particle diameter. Without intending to be limited, ejected droplets are slowed to a stop by air drag (*i.e.,* stopping distance of the ejected droplets). Ejected droplets also fall vertically due to gravity. After a short acceleration time, the droplets reach terminal velocity where the drag force equals the force of gravity. The ejected droplets may carry air along with them, which creates an entrained airstream, which aids to then carry the ejected droplets beyond the calculated stopping distance. However, increased levels of entrained air may cause the ejected droplets to flow across an impact surface (*e.g.,* an eye surface) because the entrained airflow must turn 90 degrees at such a surface. Small, ejected droplets (*e.g*., droplets having an average diameter less than about 17 microns, less than about 15 microns, *etc*.) are carried along the surface of the eye by the airstream and may not impact the surface. Contrasted to this, larger ejected droplets create less entrained air than an equivalent mass of smaller droplets, and have enough momentum to impact the surface. The ejected droplet stopping distance is a measure of this effect.

Many factors, including those described herein, can influence the desired dosage. Once the desired dosage is determined, and also if needed, desired frequency, such doses can be delivered. Frequency of dosing can vary by number of times, periodicity or both.

The term "therapeutically effective" amount refers to an amount of an active agent used to treat, ameliorate, prevent, or eliminate the identified ophthalmic condition (*e.g.,* disease or disorder), or to exhibit a detectable therapeutic or preventive effect. The effect can be detected by, for example, chemical markers, antigen levels, or time to a measurable event, such as morbidity or mortality. The precise effective amount for a subject will depend upon the subject's body weight, size, and health; the nature and extent of the condition; and the therapeutic or combination of therapeutics selected for administration. Effective amounts for a given situation can be determined by routine experimentation that is within the skill and judgment of the clinician. Any of the agents can be provided in an effective amount.

In an aspect, the concentration of an active ingredient in a medicament is measured as a percentage of the active ingredient in solution. In an aspect, the concentration of active ingredient ranges from about 0.0001% to about 5%. In another aspect, the concentration of active ingredient in a medicament ranges from about 0.05% to about 1%. In other aspects, the concentration of active ingredient ranges from about about 0.1%, about 0.2%, about 0.3%, about 0.4%, about 0.5%, about 0.75%, about 1%, about 1.5%, about 2%, about 2.5%, about 3%, about 4%, and about 5% measured as a weight percentage of the solution. However, given the lower dosing amounts afforded by the methods of the present disclosure, higher concentrations may be used depending on the intended use.

## Claims

1. A composition comprising atropine for use in a method of treatment of myopia in a child, wherein the composition comprises 0.0001% to 5% atropine by weight and is delivered to an eye of the child as a micro-dose stream of droplets by once-a-day dosing, wherein the micro-dose stream of droplets:
(i) is from 3 microliters to less than 10 microliters;
(ii) is generated via a piezoelectric droplet delivery device;
(iii) has an average ejected droplet diameter of 20 microns to 100 microns; and
(iv) has an average initial ejecting velocity of at least 3 m/s to about 4 m/s.

2. The composition for use according to claim 1, wherein the micro-dose stream of droplets delivered to the eye of the subject is 3 microliters to 7 microliters

3. The composition for use according to any one of claims 1 or 2, wherein the composition comprises atropine at a concentration of 0.05% by weight to 1% by weight.

4. The composition for use according to any one of claims 1 to 3, wherein the composition comprises atropine at a concentration of 0.1% by weight.

5. The composition for use according to any one of claims 1 to 4, wherein the micro-dose stream of droplets is delivered to the eye of the subject in less than 80 ms.

6. The composition for use according to any one of claims 1 to 5, wherein the micro-dose stream of droplets has an average ejected droplet diameter of 20 microns to 60 microns.

7. The composition for use according to any one of claims 1 to 6, wherein the piezoelectric droplet delivery device comprises an ejector mechanism, the ejector mechanism comprising a generator plate and a piezoelectric actuator, wherein the generator plate includes a plurality of openings formed through its thickness; and wherein the piezoelectric actuator is operable to directly or indirectly oscillate the generator plate, at a frequency to generate a directed stream of droplets of said low dosage volume medicament composition.

8. The composition for use according to claim 7, wherein the piezoelectric droplet delivery device further comprises an enclosure system to facilitate ejection of the stream of droplets, wherein fluid to be delivered to the eye is contained in an enclosure which holds the fluid to be dispensed in a chamber defined by the enclosure, and wherein the enclosure holds the composition in proximity to the openings of the generator plate of the ejector mechanism.

9. The composition for use according to claim 8, wherein the piezoelectric droplet delivery device further comprises a lip positioned adjacent to the generator plate; wherein the piezoelectric actuator vibrates the generator plate by an amplitude which is less than an average separation distance between the lip and the generator plate or less than a minimum separation distance between the lip and the generator plate.

10. The composition for use according to claim 9, wherein the piezoelectric droplet delivery device further comprises a lip biased against the generator plate and configured to exert a force of no more than 3 gram-f on the vibrating element measured in the direction of a central axis of the vibrating element.

11. The composition for use according to any preceding claim, wherein the piezoelectric droplet delivery device includes smart electronics that are designed to track when a patient administers treatment.

## Patentansprüche

1. Zusammensetzung, die Atropin umfasst, zur Verwendung in einem Verfahren zur Behandlung von Myopie bei einem Kind, wobei die Zusammensetzung 0,0001 bis 5 Gew.-% Atropin umfasst und als Mikrodosisstrom von Tröpfen in einer Dosierung von einmal täglich an ein Auge des Kindes verabreicht wird, wobei der Mikrodosisstrom von Tröpfchen:
(i) 3 µl bis weniger als 10 µl umfasst;
(ii) mithilfe einer piezoelektrischen Tröpfchenabgabevorrichtung erzeugt wird;
(iii) einen durchschnittlichen Durchmesser der ausgestoßenen Tröpfchen von 20 µm bis 100 µm aufweist; und
(iv) eine durchschnittliche anfängliche Ausstoßgeschwindigkeit von zumindest 3 m/s bis etwa 4 m/s aufweist.

2. Zusammensetzung zur Verwendung nach Anspruch 1, wobei der Mikrodosisstrom von Tröpfchen, der dem Auge des Individuums verabreicht wird, 3 µl bis 7 µl umfasst.

3. Zusammensetzung zur Verwendung nach einem der Ansprüche 1 oder 2, wobei die Zusammensetzung Atropin in einer Konzentration von 0,05 bis 1 Gew.-% umfasst.

4. Zusammensetzung zur Verwendung nach einem der Ansprüche 1 bis 3, wobei die Zusammensetzung Atropin in einer Konzentration von 0,1 Gew.-% umfasst.

5. Zusammensetzung zur Verwendung nach einem der Ansprüche 1 bis 4, wobei der Mikrodosisstrom von Tröpfchen in weniger als 80 ms an das Auge des Individuums verabreicht wird.

6. Zusammensetzung zur Verwendung nach einem der Ansprüche 1 bis 5, wobei der Mikrodosisstrom von Tröpfchen einen durchschnittlichen Durchmesser der ausgestoßenen Tröpfchen von 20 µm bis 60 µm aufweist.

7. Zusammensetzung zur Verwendung nach einem der Ansprüche 1 bis 6, wobei die piezoelektrische Tröpfchenabgabevorrichtung einen Ausstoßmechanismus umfasst, wobei der Ausstoßmechanismus eine Erzeugungsplatte und einen piezoelektrischen Aktuator umfasst, wobei die Erzeugungsplatte eine Vielzahl von Öffnungen umfasst, die durch ihre Dicke hindurch ausgebildet sind; und wobei der piezoelektrische Aktuator betreibbar ist, um die Erzeugungsplatte direkt oder indirekt mit einer Frequenz zu oszillieren, um einen gerichteten Tröpfchenstrom der Medikamentenzusammensetzung mit niedrigem Dosierungsvolumen zu erzeugen.

8. Zusammensetzung zur Verwendung nach Anspruch 7, wobei die piezoelektrische Tröpfchenabgabevorrichtung weiters ein Einfassungssystem umfasst, um den Ausstoß des Tröpfchenstroms zu erleichtern, wobei Flüssigkeit, die an das Auge abgegeben werden soll, in einer Einfassung enthalten ist, welche die abzugebende Flüssigkeit in einer Kammer enthält, die durch die Einfassung definiert ist, und wobei die Einfassung die Zusammensetzung nahe bei den Öffnungen der Erzeugungsplatte des Ausstoßmechanismus hält.

9. Zusammensetzung zur Verwendung nach Anspruch 8, wobei die piezoelektrische Tröpfchenabgabevorrichtung weiters eine Lippe umfasst, die neben der Erzeugungsplatte positioniert ist; wobei der piezoelektrische Aktuator die Erzeugungsplatte mit einer Amplitude vibriert, die kleiner als der durchschnittliche Trennungsabstand zwischen der Lippe und der Erzeugungsplatte oder kleiner als der minimale Trennungsabstand zwischen der Lippe und der Erzeugungsplatte ist.

10. Zusammensetzung zur Verwendung nach Anspruch 9, wobei die piezoelektrische Tröpfchenabgabevorrichtung weiters eine Lippe umfasst, die gegen die Erzeugungsplatte vorgespannt und konfiguriert ist, um eine Kraft von nicht mehr als 3 Pond, gemessen in Richtung der Zentralachse des Vibrationselements, auf das Vibrationselement auszuüben.

11. Zusammensetzung zur Verwendung nach einem der vorangegangenen Ansprüche, wobei die piezoelektrische Tröpfchenabgabevorrichtung intelligente Elektronik umfasst, die dazu ausgelegt ist nachzuverfolgen, wann ein Patient eine Behandlung verabreicht.

## Revendications

1. Composition comprenant de l'atropine à utiliser dans un procédé de traitement de la myopie chez un enfant, dans laquelle la composition comprend de 0,0001 % à 5 % d'atropine en poids et est distribuée à un œil de l'enfant sous la forme d'un flux de gouttelettes micro-dosé par dosage une fois par jour, dans laquelle le flux de gouttelettes micro-dosé :
(i) est de 3 microlitres à moins de 10 microlitres ;
(ii) est générée via un dispositif de distribution de gouttelettes piézoélectrique ;
(iii) présente un diamètre moyen de gouttelettes éjectées de 20 microns à 100 microns ; et
(iv) présente une vitesse d'éjection initiale moyenne d'au moins 3 m/s à environ 4 m/s.

2. Composition à utiliser selon la revendication 1, dans laquelle le flux de gouttelettes micro-dosé délivré à l'œil du sujet est de 3 microlitres à 7 microlitres.

3. Composition à utiliser selon l'une quelconque des revendications 1 ou 2, dans laquelle la composition comprend de l'atropine à une concentration de 0,05 % en poids à 1 % en poids.

4. Composition à utiliser selon l'une quelconque des revendications 1 à 3, dans laquelle la composition comprend de l'atropine à une concentration de 0,1 % en poids.

5. Composition à utiliser selon l'une quelconque des revendications 1 à 4, dans laquelle le flux de gouttelettes micro-dosé est distribué à l'œil du sujet en moins de 80 ms.

6. Composition à utiliser selon l'une quelconque des revendications 1 à 5, dans laquelle le flux de gouttelettes micro-dosé présente un diamètre de gouttelettes éjectées moyen de 20 microns à 60 microns.

7. Composition à utiliser selon l'une quelconque des revendications 1 à 6, dans laquelle le dispositif de distribution de gouttelettes piézoélectrique comprend un mécanisme éjecteur, le mécanisme éjecteur comprenant une plaque de génération et un actionneur piézoélectrique, dans laquelle la plaque de génération inclut une pluralité d'ouvertures formées à travers son épaisseur ; et dans laquelle l'actionneur piézoélectrique peut fonctionner pour faire osciller directement ou indirectement la plaque de génération, à une fréquence pour générer un flux de gouttelettes dirigé de ladite composition médicamenteuse à faible volume de dosage.

8. Composition à utiliser selon la revendication 7, dans laquelle le dispositif de distribution de gouttelettes piézoélectrique comprend en outre un système d'enceinte pour faciliter une éjection du flux de gouttelettes, dans laquelle le fluide à distribuer à l'œil est contenu dans une enceinte qui contient le fluide à distribuer dans une chambre définie par l'enceinte, et dans laquelle l'enceinte contient la composition à proximité des ouvertures de la plaque de génération du mécanisme d'éjection.

9. Composition à utiliser selon la revendication 8, dans laquelle le dispositif de distribution de gouttelettes piézoélectrique comprend en outre une lèvre positionnée adjacente à la plaque de génération ; dans laquelle l'actionneur piézoélectrique fait vibrer la plaque de génération d'une amplitude qui est inférieure à une distance de séparation moyenne entre la lèvre et la plaque de génération, ou inférieure à une distance de séparation minimale entre la lèvre et la plaque de génération.

10. Composition à utiliser selon la revendication 9, dans laquelle le dispositif de distribution de gouttelettes piézoélectrique comprend en outre une lèvre sollicitée contre la plaque de génération et configurée pour exercer une force de pas plus de 3 grammes-f sur l'élément vibrant mesurée dans la direction d'un axe central de l'élément vibrant.

11. Composition à utiliser selon l'une quelconque des revendications précédentes, dans laquelle le dispositif de distribution de gouttelettes piézoélectrique comprend une électronique intelligente qui est conçue pour suivre le moment où un patient administre un traitement.
